# EUROPEAN PATENT APPLICATION

(11) **EP 3 466 361 A1**
(43) Date of publication of application: **10.04.2019**
(21) Application number: 16904076.3
(22) Date of filing: 03.06.2016
(51) Int. Cl.: A61B 17/94, A61B 18/00, A61B 90/00

(54) **MEDICAL DEVICE**

(71) Applicant: Olympus Corporation, Hachioji-shi Tokyo 192-8507 (JP)
(72) Inventor: KIKUCHI, Chisato, Hachioji-shi Tokyo 192-8507 (JP)
(74) Representative: Gunzelmann, Rainer
(86) International application number: PCT/JP2016/066644
(87) International publication number: WO 2017/208451

(57) **Abstract**

A medical device (1) of the present invention is provided with: a long, thin elongated portion (2) that can be inserted into one of two cavities that are separated by a body wall; a sheet portion (3) that is provided at a distal end of the elongated portion (2) and that covers a surface of the body wall; an attaching portion (6) that is provided around the entire circumference of the periphery of the sheet portion (3) and that is brought into close contact with a surface of the body wall; and a notifying portion (5) that is provided on the sheet portion (3) and that notifies a surgeon about the fact that an incision has been made in the body wall from the other cavity into the one cavity on the basis of exposure of the sheet portion (3) to the other cavity from the incision site in the body wall.

## Description

### {Technical Field}

The present invention relates to a medical device.

### {Background Art}

When treating the lining of the digestive tract, it is required to prevent the exterior of the digestive tract from being polluted by the contents of the digestive tract. Therefore, methods for treating the lining while keeping the interior and the exterior of the digestive tract isolated from each other have been proposed (for example, see Patent Literature 1 and Non-Patent Literature 1).

The equipment described in Patent Literature 1 is provided with a capping member that expands like a sheet inside the digestive tract, thus blocking an opening formed in the digestive-tract wall. With the surgical method proposed in Non-Patent Literature 1, in order to remove a tumor formed in the gastric mucosa without opening up the interior of the stomach, only the outer portion of the stomach wall is excised from outside the stomach, the excision site is subsequently sutured, and then, the remaining portion of the stomach wall is excised from inside the stomach.

### {Citation List}

### {Patent Literature}

{PTL 1} Japanese Translation of PCT International Application, Publication No. 2009-540963

### {Non-Patent Literature}

{NPL 1} University of Tokyo Gastroenterology Digestive Tract Group, "NEWS (Non-exposed Endoscopic Wall-inversion Surgery)", [online], [accessed on March 9, 2016], Internet <URL: http://www.todai-shoukakan.com/cont3/13.html>

### {Summary of Invention}

### {Technical Problem}

However, because the capping member described in Patent Literature 1 is formed of a collagenous material, it tends to be damaged by a high-frequency treatment tool or a sharp treatment tool that is used to remove an affected portion, and, in addition, it is difficult for a surgeon to recognize damage to the capping member. Because of this, there is a problem in that it is difficult to reliably keep the interior and the exterior of the digestive tract isolated from each other while treatment is being performed. With the surgical method described in Non-Patent Literature 1, because marking, cutting, and suturing of the affected portion are performed from outside the digestive tract and, subsequently, cutting and suturing of the affected portion are performed from inside the digestive tract, there is a problem in that the amount of time required to perform treatment is increased.

The present invention has been conceived in light of the above-described problem, and an object thereof is to provide a medical device with which it is possible to treat a body wall while reliably keeping cavities on either side of the body wall isolated from each other, and with which it is also possible to reduce the amount of time required to perform treatment.

### {Solution to Problem}

In order to achieve the above-described object, the present invention provides the following solutions.

An aspect of the present invention is a medical device that is used to make an incision in a body wall that separates two cavities in a body by making the incision from the one cavity, the medical device including: a long, thin elongated portion that can be inserted into the other cavity; a sheet portion that is provided at a distal end of the elongated portion, that is disposed facing a surface of the body wall, and that covers the surface of the body wall; an attaching portion that is provided around the entire circumference of the periphery of the sheet portion and that is brought into close contact with the surface of the body wall; and a notifying portion that is provided on the sheet portion and that notifies a surgeon about the fact that an incision has been made in the body wall from the one cavity into the other one cavity on the basis of exposure of the sheet portion to the one cavity from the incision site in the body wall.

With this aspect, the sheet portion disposed in the other cavity is disposed facing the body wall so as to cover an affected portion in the body wall, and the attaching portion is brought into close contact with the surface of the body wall. By doing so, the affected portion is isolated in the other cavity. Next, an incision is made in the affected portion by using a treatment tool such as a scalpel that is inserted into the one cavity and the incision site is sutured, and thus, it is possible to treat the affected portion.

In this case, when an incision is made in the body wall so as to reach into the other cavity, the sheet portion that covers the affected portion from the other cavity is exposed to the one cavity. At this time, because the area surrounding the sheet portion is sealed by the attaching portion that is in close contact with the body wall, the two cavities do not communicate with each other via the incision site. The notifying portion notifies the surgeon about the fact that the body wall has been penetrated on the basis of exposure of the sheet portion to the one cavity. Because the surgeon can recognize that the body wall has been penetrated on the basis of the notification issued by the notifying portion, it is possible to avoid damage on the sheet portion caused by the treatment tool. By doing so, it is possible to reliably continue to isolate the two cavities on either side of the cavity wall from each other even after the body wall has been penetrated by the incision. Furthermore, because treatment of the affected portion may be performed only from the one cavity, it is also possible to reduce the amount of time required to perform treatment.

In the above-described aspect, the sheet portion can be deformed between an open form in which the sheet portion is expanded in a direction that intersects a longitudinal direction of the elongated portion and a closed form in which the sheet portion is folded so as to be placed along the longitudinal direction of the elongated portion, and an opening/closing mechanism that causes the sheet portion to be deformed between the open form and the closed form may be provided.

By doing so, because the radial size of the medical device is decreased by placing the sheet portion in the closed form, the medical device can be inserted into and removed from a cavity via a narrow space such as a channel of the endoscope.

In the above-described aspect, the notifying portion may be provided with a color layer that is provided in a surface of the sheet portion, which is disposed facing the surface of the body wall, and that has a color that is complementary to the color of the body wall or a color that is close to the complementary color.

By doing so, it is possible to notify the surgeon about penetration of the body wall by means of the color of the color layer exposed to the one cavity via the incision site.

In the above-described aspect, the attaching portion may protrude farther than a surface, which is disposed facing a surface of the body wall, of the sheet portion does.

By doing so, it is possible to isolate the affected portion in the other cavity while ensuring a sufficient distance between the surface of the body wall and the sheet portion.

In the above-described aspect, the attaching portion may be formed of a biological adhesive that is applied around the entire circumference in the periphery of the sheet portion.

By doing so, it is possible to secure the sheet portion to the surface of the body wall by means of the adhesive force of the biological adhesive.

In the above-described aspect, the attaching portion may be provided with a suction port provided around a circumferential direction thereof, and a suction device that is disposed at a proximal end of the elongated portion and that generates a suction force and a suction pipe that connects the suction port and the suction device via the elongated portion may be provided.

By doing so, it is possible to make the attaching portion adhere to the body wall by generating the suction force at the suction port via the interior of the suction pipe by means of the suction device. In addition, by stopping sucking by the suction device, it is possible to easily detach the attaching portion from the body wall.

In the above-described aspect, the notifying portion may be provided with a reflector that is provided in a surface of the sheet portion, which is disposed facing a surface of the body wall, and that reflects light.

By doing so, it is possible to notify the surgeon about penetration of the body wall by means of the light reflected by the reflector toward the one cavity.

In the above-described aspect, the notifying portion may be provided with a light-emitting body that is provided on a surface of the sheet portion, which is disposed facing a surface of the body wall, and that emits light.

By doing so, it is possible to notify the surgeon about penetration of the body wall by means of the light emitted from the light-emitting body toward the one cavity.

In the above-described aspect, the notifying portion may be provided with: a contact detecting means that is provided on the sheet portion and that detects contact with a treatment tool used to make an incision in the body wall; and a recognizing means for visually or aurally making the surgeon recognize that contact with the treatment tool has been detected by the contact detecting means.

By doing so, when the treatment tool that has penetrated the body wall comes into contact with the sheet portion, this contact is detected by the contact detecting means, and the recognizing means visually or aurally notifies the surgeon about the penetration. By doing so, it is possible to more reliably prevent the sheet portion from being damaged by the treatment tool.

In the above-described aspect, the sheet portion may be provided with an insulating layer possessing electrical insulating properties.

By doing so, because an incision is not made in the insulating layer by an electric treatment tool such as an electric scalpel, it is possible to more reliably prevent, by means of the insulating layer, the sheet portion from being damaged by the electric treatment tool.

In the above-described aspect, the sheet portion may be provided with a conductive layer possessing conductivity, and an earth line that connects the conductive layer to earth via the elongated portion may be provided.

By doing so, because a current output from the electric treatment tool when the electric treatment tool, such as an electric scalpel, that has penetrated the body wall comes into contact with the conductive layer flows to the earth via the earth line, an incision is not made in the conductive layer by the electric treatment tool. By doing so, it is possible to more reliably prevent, by means of the insulating layer, the sheet portion from being damaged by the electric treatment tool.

### {Advantageous Effects of Invention}

The present invention affords an advantage in that it is possible to treat a body wall while keeping cavities on either side of the body wall isolated from each other, and in that it is also possible to reduce the amount of time required to perform treatment.

### {Brief Description of Drawings}

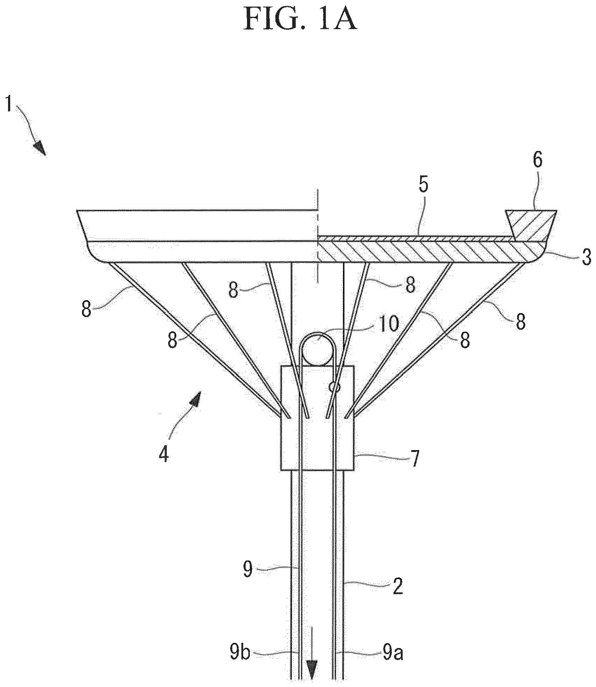

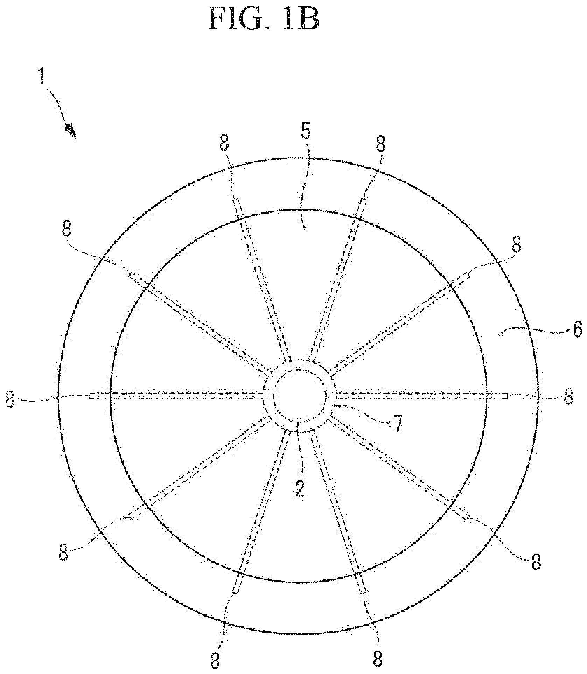

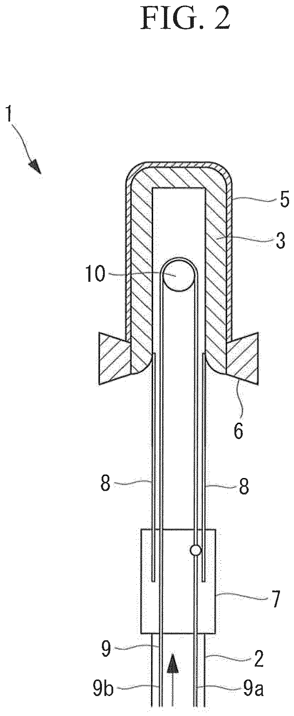

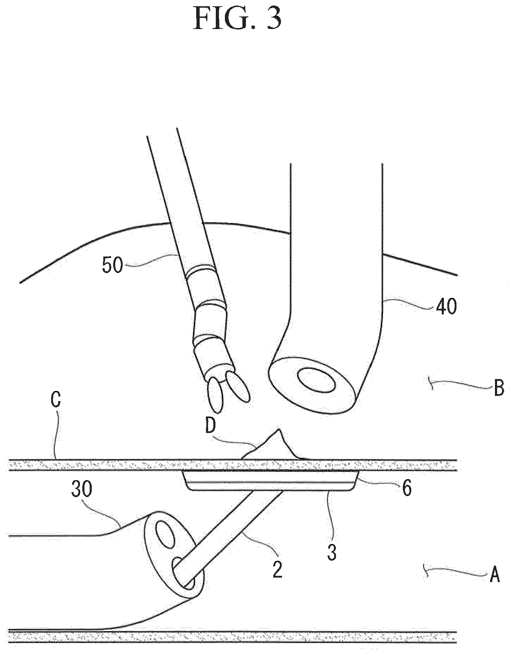

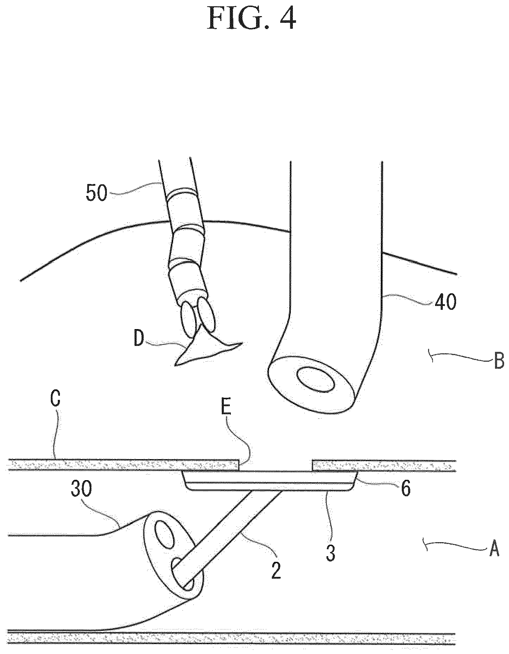

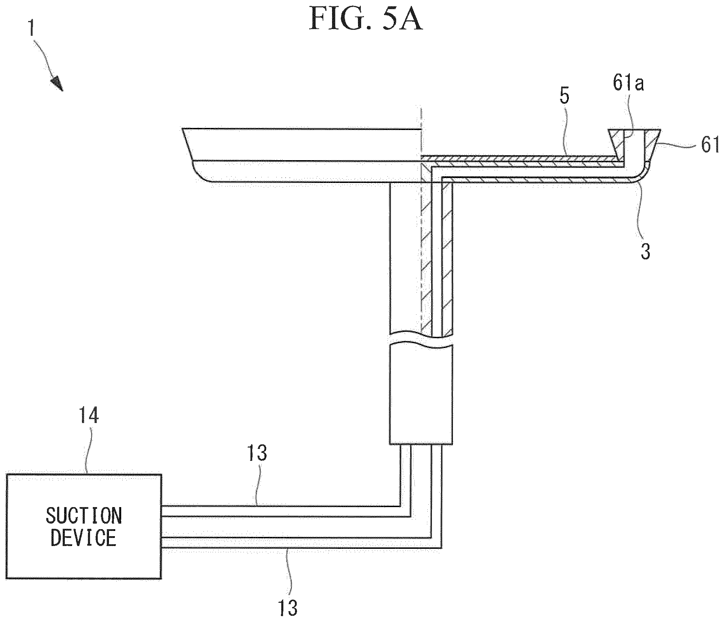

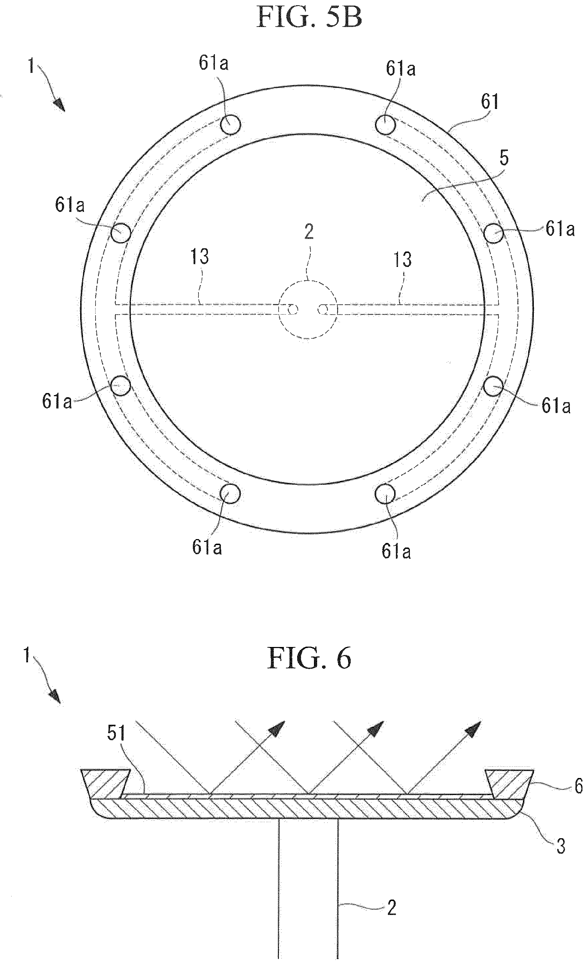

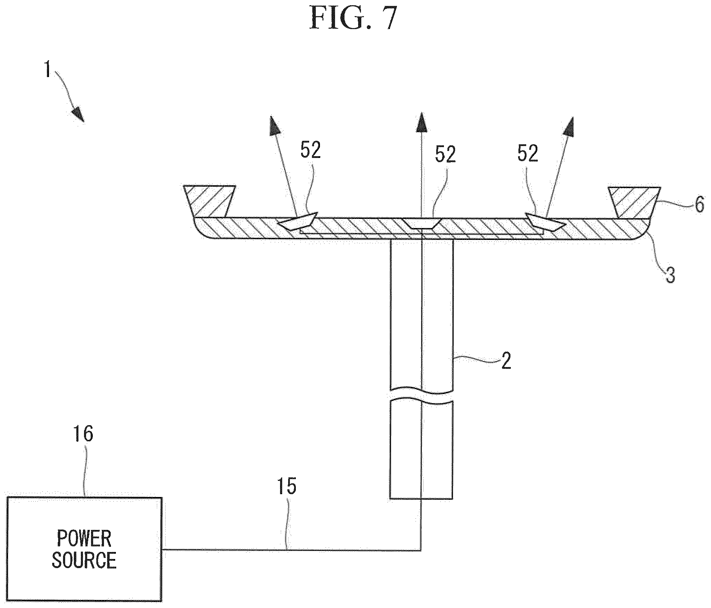

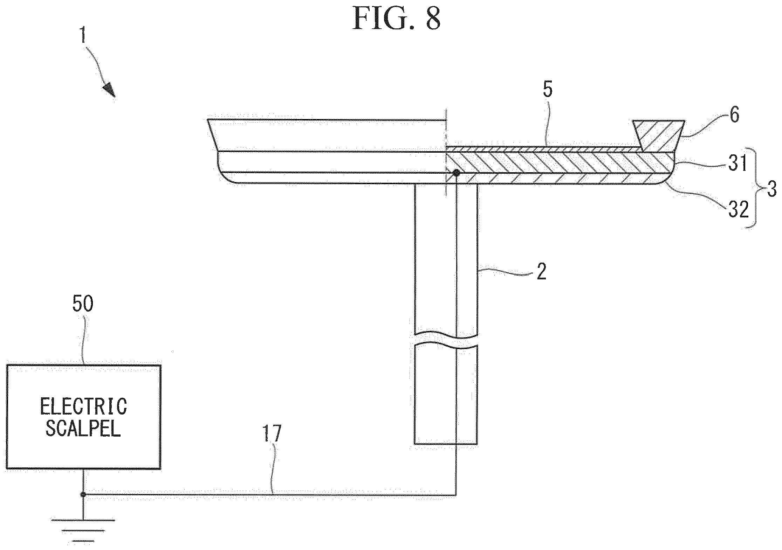

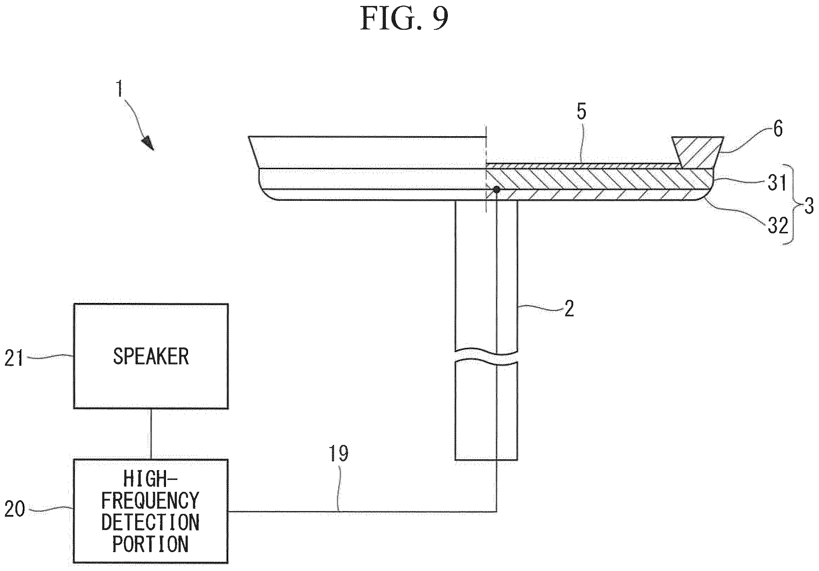

{Fig. 1A} Fig. 1A is a configuration diagram of a medical device according to an embodiment of the present invention in an open form.
{Fig. 1B} Fig. 1B is a front view of the medical device in Fig. 1A viewed from a distal-end side.
{Fig. 2} Fig. 2 is a configuration diagram of the medical device in Fig. 1A in a closed form.
{Fig. 3} Fig. 3 is a diagram for explaining the manner in which the medical device in Fig. 1A is used.
{Fig. 4} Fig. 4 is a diagram for explaining the manner in which the medical device in Fig. 1A is used.
{Fig. 5A} Fig. 5A is a configuration diagram of a modification of the medical device in Fig. 1A.
{Fig. 5B} Fig. 5B is a front view of the medical device in Fig. 5A viewed from a distal-end side.
{Fig. 6} Fig. 6 is a configuration diagram of another modification of the medical device in Fig. 1A.
{Fig. 7} Fig. 7 is a configuration diagram of another modification of the medical device in Fig. 1A.
{Fig. 8} Fig. 8 is a configuration diagram of another modification of the medical device in Fig. 1A.
{Fig. 9} Fig. 9 is a configuration diagram of another modification of the medical device in Fig. 1A.

### {Description of Embodiment}

A medical device 1 according to an embodiment of the present invention will be described below with reference to the drawings.

As shown in Figs. 1A and 1B, the medical device 1 according to this embodiment is provided with: a long, thin elongated portion 2; a sheet portion 3 that is provided at a distal end of the elongated portion 2 and that can be expanded and contracted in the radial direction with respect to the elongated portion 2; a opening/closing mechanism 4 that causes the sheet portion 3 to be deformed; and a notifying portion 5 and an attaching portion 6 that are provided on the sheet portion 3.

The medical device 1 is used by being inserted into a channel of a lumen endoscope. The lumen endoscope is a small-diameter, flexible endoscope to be used in a lumen such as the stomach or the intestine.

The elongated portion 2 has a diameter that allows insertion thereof into the channel of the lumen endoscope. In addition, the elongated portion 2 has flexibility that allows bending thereof in conformity to bending of the lumen endoscope in the body.

The sheet portion 3 is formed of a circular sheet-like member that is formed of a resin material having flexibility, such as silicone rubber, urethane rubber, or vinyl chloride. The sheet portion 3 has a distal-end surface and a proximal-end surface that face each other in the thickness direction, and is joined with the distal end of the elongated portion 2 at substantially the center of the proximal-end surface. Such a sheet portion 3 can be deformed like the canopy of an umbrella between the open form shown in Fig. 1A and the closed form shown in Fig. 2. The open form is a form in which the sheet portion 3 is expanded to be flat in a direction that intersects the longitudinal direction of the elongated portion 2. The closed form is a form in which the sheet portion 3 is folded so as to be placed along the longitudinal direction of the elongated portion 2.

The opening/closing mechanism 4 is provided with: a sliding portion 7 that is provided in the elongated portion 2 so as to be movable along the longitudinal direction of the elongated portion 2; a plurality of ribs 8, each of which have two ends thereof connected to the proximal-end surface of the sheet portion 3 and the sliding portion 7; and a wire 9 that drives the sliding portion 7 along the longitudinal direction of the elongated portion 2.

The plurality of ribs 8 are arrayed in a radiating manner centered on the elongated portion 2 with spaces therebetween in the circumferential direction of the elongated portion 2. The distal ends of the individual ribs 8 are secured to the periphery of the proximal-end surface of the sheet portion 3.

The elongated portion 2 is provided with a shaft portion 10 that extends in the radial direction of the elongated portion 2 at a position that is closer to the distal end than the sliding portion 7 is. The wire 9 is threaded around the shaft portion 10, and two end portions 9a and 9b of the wire 9 are disposed at the proximal end of the elongated portion 2. By doing so, the two end portions 9a and 9b of the wire 9 are configured so as to be moved in mutually opposing directions. In addition, an intermediate position of the wire 9 is secured to the sliding portion 7. Therefore, by pulling the one end portion 9a of the wire 9 toward the proximal end, the sliding portion 7 is moved toward the proximal end, and thus, the ribs 8 pull the sheet portion 3 into the closed form. On the other hand, by pulling the other end portion 9b of the wire 9 toward the proximal end, the sliding portion 7 is moved toward the distal end, and thus, the ribs 8 push and expand the sheet portion 3 into the open form. In order to assist the deformation of the sheet portion 3 from the closed form to the open form, the ribs 8 may possess an elastic force that biases the sheet portion 3 toward the distal end. For example, the ribs 8 may be formed of elastic members such as springs.

The notifying portion 5 is formed of a color layer (hereinafter also referred to as color layer 5) provided on the distal-end surface of the sheet portion 3. The color layer 5 covers the entire distal-end surface of the sheet portion 3, excluding the attaching portion 6. On the surface of the color layer 5, water repellent treatment may be applied in order to prevent attachment of a liquid such as blood or the like.

It is preferable that the color layer 5 have a color that is different from that of the body wall so as to be conspicuous in the body, and have a color that is complementary to the colors of the body wall and blood or a color close to a complementary color. Furthermore, it is preferable that the color of the color layer 5 be a color with which an afterimage is unlikely to be generated. Examples of such color include green and blue. Although the color of the color layer 5 may be uniform over the entirety thereof, a pattern (for example, a stripe pattern or a checkered pattern) formed of a plurality of colors may be applied to the color layer 5 in order to further increase the visibility of the color layer 5 in the body.

The attaching portion 6 is formed of a biological adhesive, such as a fibrin glue, that is applied to the distal-end surface of the sheet portion 3. The biological adhesive is applied, in an annular manner, around the entire circumference of the periphery of the surface of the sheet portion 3, and protrudes farther than the distal-end surface of the sheet portion 3 in the thickness direction of the sheet portion 3. The amount by which the attaching portion 6 protrudes from the distal-end surface of the sheet portion 3 is designed so that the outer size of the attaching portion 6 in the radial direction of the elongated portion 2 in the closed form of the sheet portion 3 becomes smaller than the inner size of the channel of a lumen endoscope 30. The biological adhesive is a thermoresponsive adhesive that exhibits adhesiveness by being heated to the body temperature. Therefore, the sheet portion 3 is secured to the body wall by means of the adhesiveness that the attaching portion 6 exhibits by being in contact with the body wall.

Next, the operation of the thus-configured medical device 1 will be described.

As shown in Fig. 3, the medical device 1 according to this embodiment is used in a surgery for excising, by using the lumen endoscope 30 inserted into a lumen A and a body cavity endoscope 40 inserted into a body cavity B, an affected portion D, such as a tumor, formed in a wall C of the lumen A that separates the lumen A and the body cavity B. The lumen A is, for example, the stomach or the intestine, and the body cavity B is, for example, the abdominal cavity.

A surgeon sets the sheet portion 3 to the closed form by pulling the one end portion 9a of the wire 9, which extends from the proximal end of the elongated portion 2, toward the proximal end, and places the sheet portion 3 in the vicinity of the affected portion D via the channel of the lumen endoscope 30 inserted into the lumen A. Next, the surgeon expands the sheet portion 3 into the open form by pulling the other end portion 9b of the wire 9.

Next, while observing the affected portion D by using the lumen endoscope 30 from inside the lumen A, the surgeon places the sheet portion 3 facing the surface of the wall C so that the entire affected portion D is covered by the sheet portion 3, thus bringing the attaching portion 6 into close contact with the wall C. The biological adhesive of the attaching portion 6 heated by the body temperature exhibits adhesiveness, thus securing the sheet portion 3 to the wall C. At this time, the space between the wall C and the sheet portion 3 is sealed by the attaching portion 6 that is provided around the entire circumference of the periphery of the sheet portion 3, and thus, the affected portion D is isolated from the internal space in the lumen A.

Next, as shown in Fig. 4, while observing the affected portion D from outside the lumen A by using the body cavity endoscope 40 inserted into the body cavity B, the affected portion D is excised from the wall C by making an incision in the area surrounding the affected portion D by using a treatment tool 50 (for example, a scalpel, a high-frequency knife, an ultrasonic knife or scissors) inserted into the body cavity B. Because a hole E formed in the wall C due to excision of the affected portion D is blocked by the sheet portion 3 and the attaching portion 6, the lumen A does not communicate with the body cavity B via the hole E. After excising the affected portion D, the surgeon completes treatment of the wall C by suturing the hole E.

In this case, with this embodiment, when the incision is made in the wall C from the body cavity B into the lumen A, the color layer 5 that covers the wall C on the lumen A is exposed to the body cavity B via the incision site, and thus, the color layer 5 is observed in the incision site in the image captured by the body cavity endoscope 40. When the color layer 5, which has a conspicuous color with respect to the wall C, appears in the image in this way, the surgeon receives a visual notification that the wall C has been penetrated into the lumen A. Therefore, the surgeon can quickly and easily recognize penetration of the wall C, and thus, it is possible to appropriately manipulate the treatment tool 50 so that the sheet portion 3 is not damaged by the treatment tool 50. By doing so, there is an advantage in that, while treating the wall C, it is possible to reliably keep the lumen A and the body cavity B isolated from each other by means of the sheet portion 3 and the attaching portion 6 that block the incision site from the lumen A. Furthermore, because excision of the affected portion D may be performed only from the body cavity B, there is an advantage in that it is possible to reduce the amount of time required to perform treatment.

In this embodiment, it is preferable that the sheet portion 3 have a thickness of 2 mm or greater. With the sheet portion 3 having a thickness of 2 mm or greater, even if the treatment tool 50 comes into contact with the sheet portion 3, it is possible to more reliably prevent the sheet portion 3 from being penetrated by the treatment tool 50.

In this embodiment, although the attaching portion 6 is formed of the thermoresponsive adhesive, alternatively, the attaching portion 6 may be formed of a moisture-reactive biological adhesive that exhibits adhesiveness via a reaction with water. By doing so also, it is possible to secure the sheet portion 3 to the wall C by causing the attaching portion 6 to exhibit adhesiveness via contact with the wall C.

Alternatively, as shown in Figs. 5A and 5B, an attaching portion 61 may be configured to be secured to the wall C by means of adhesion. The attaching portion 61 is provided around the entire circumference of the periphery of the distal-end surface of the sheet portion 3, and is formed of an annular member that protrudes from the distal-end surface of the sheet portion 3. As shown in Fig. 5B, a plurality of suction ports 61a, which are arranged with spacings therebetween in the circumferential direction, are provided at the distal-end surface of the attaching portion 61.

The plurality of suction ports 61a are connected, via suction pipes 13 that pass through the interior of the elongated portion 2, to a suction device 14 (for example, a pump or a syringe) that is disposed at the proximal end of the elongated portion 2 and that generates a suction force. The distal-end surface of the attaching portion 6 adheres to the wall C due to the suction force generated by the suction device 14. Although Figs. 5A and 5B show the plurality of suction ports 61a that are arranged with spacings therebetween in the circumferential direction, the suction ports may have a circular shape or an arc-like shape that extends around the circumferential direction of the attaching portion 6.

Note that, in Figs. 5A to 9, the illustration of the opening/closing mechanism 4 is omitted in order to simplify the drawings.

In this embodiment, although the notifying portion is formed of the color layer 5 that has a color that is different from that of the wall C, alternatively, as shown in Fig. 6, the notifying portion may be formed of a reflector 51 that covers the entire distal-end surface of the sheet portion 3 and that reflects light. Illumination light that is radiated onto the wall C from the distal-end surface of the body cavity endoscope 40 is reflected toward the body cavity B by the reflector 51 exposed from the incision site, and the reflected light is observed in the image captured by the body cavity endoscope 40. By doing so, it is possible to visually notify the surgeon about penetration of the wall C.

The reflector 51 may be a metal film that is formed by coating the distal-end surface of the sheet portion 3, or may be a metal foil attached to the distal-end surface of the sheet portion 3. Alternatively, the reflector 51 may be a metal sheet having a mirror-finish surface. The surface of the reflector 51 may be an irregular surface oriented in various directions so as to reflect light in various directions. In addition, the reflector 51 may have a pattern (for example, a stripe pattern or a checkered pattern) formed of reflective regions having high optical reflectance and nonreflective regions having low optical reflectance.

As shown in Fig. 7, the notifying portion may be formed of light-emitting bodies 52 that are provided at the distal-end surface of the sheet portion 3 and that emit light. By doing so also, light emitted into the body cavity B from the light-emitting bodies 52 exposed from the incision site is observed in the image, and thus, it is possible to visually notify the surgeon about penetration of the wall C.

The light-emitting bodies 52 are constituted of, for example, a plurality of LEDs or organic EL devices that are arrayed at the distal-end surface of the sheet portion 3. The light-emitting bodies 52 are connected to a power source 16 that is disposed at the proximal end of the elongated portion 2 via an electric wire 15 that passes through the interior of the elongated portion 2 so that power is supplied from the power source 16 via the electric wire 15. It is preferable that the plurality of light-emitting bodies 52 be disposed so as to emit light in various directions so that light coming from the light-emitting bodies 52 can be recognized regardless of the direction from which the sheet portion 3 is observed. It is preferable that the color of the light emitted by the light-emitting bodies 52 be, as with the color of the color layer 5, a color that is complementary to the colors of the wall C and blood or a color that is close to a complementary color, and with which an afterimage is unlikely to be generated. The light may flicker or the color of the light may be changed among multiple colors so that the surgeon can more easily recognize the light coming from the light-emitting bodies 52.

In this embodiment, although the sheet portion 3 is constituted of a single layer formed of a resin material, alternatively, as shown in Fig. 8, the sheet portion 3 may include a conductive layer 31 possessing conductivity and an insulating layer 32 that covers the proximal-end surface of the conductive layer 31 and that has electrical insulating properties. It is preferable that the conductive layer 31 and the insulating layer 32 be formed over the entire region of the sheet portion 3 in the surface direction of the sheet portion 3.

The conductive layer 31 is connected to an earth of the electric scalpel 50 via an earth line 17 that passes through the interior of the elongated portion 2. Because a current flows from the electric scalpel 50 to the earth via the conductive layer 31 and the earth line 17 when the electric scalpel 50 penetrates the notifying portion 5 and comes into contact with the conductive layer 31, an incision is not made in the conductive layer 31 by the electric scalpel 50. By doing so, it is possible to more reliably prevent the sheet portion 3 from being damaged.

Furthermore, because an incision is also not made in the insulating layer 32 by the electric scalpel 50, it is possible to more reliably prevent the sheet portion 3 from being damaged.

In this embodiment, although the surgeon is visually made to recognize, by means of an image, the notifying portion 5 that is exposed to the body cavity B via the incision site, whereby the surgeon is notified about penetration of the wall C, alternatively, penetration of the wall C may be notified by using other means.

For example, as shown in Fig. 9, the notifying portion may be provided with: the conductive layer (contact detecting means) 31; a high-frequency detection portion (contact detecting means) 20 that is connected to the conductive layer 31 via an electric wire 19 that passes through the interior of the elongated portion 2; and a speaker (recognizing means) 21 that is connected to the high-frequency detection portion 20.

When the electric scalpel 50 comes into contact with the conductive layer 31 of the sheet portion 3, which is exposed to the body cavity B from the incision site in the wall C, a high-frequency electric current output from the electric scalpel 50 is detected by the high-frequency detection portion 20 via the electric wire 19. The high-frequency detection portion 20 causes the speaker 21 to output a sound when the high-frequency electric current is detected. By doing so, it is possible to aurally notify the surgeon about penetration of the wall C, thus making him/her recognize penetration of the wall C. The surgeon may be made to recognize penetration of the wall C by using a visual means (for example, a warning display that is displayed on a display together with the image captured by the body cavity endoscope 40) instead of the sound.

In this embodiment, although a case in which the wall C that separates the lumen A and the body cavity B is treated has been described, the medical device 1 can be used to treat an arbitrary body wall that separates two cavities (for example, the diaphragm that separates the abdominal cavity and the chest cavity).

### {Reference Signs List}

1 medical device
2 elongated portion
3 sheet portion
31 conductive layer
32 insulating layer
4 opening/closing mechanism
5 color layer (notifying portion)
51 reflector (notifying portion)
52 light-emitting body (notifying portion)
6, 61 attaching portion
61a suction port
13 suction pipe
14 suction device
17 earth line
20 high-frequency detection portion (notifying portion, contact detecting means)
21 speaker (notifying portion, recognizing means)
A lumen (cavity)
B body cavity (cavity)
C lumen wall (body wall)

## Claims

1. A medical device that is used to make an incision in a body wall that separates two cavities in a body by making the incision from the one cavity, the medical device comprising:
a long, thin elongated portion that can be inserted into the other cavity;
a sheet portion that is provided at a distal end of the elongated portion, that is disposed facing a surface of the body wall, and that covers the surface of the body wall;
an attaching portion that is provided around the entire circumference of the periphery of the sheet portion and that is brought into close contact with the surface of the body wall; and
a notifying portion that is provided on the sheet portion and that notifies a surgeon about the fact that an incision has been made in the body wall from the one cavity into the other one cavity on the basis of exposure of the sheet portion to the one cavity from the incision site in the body wall.

2. A medical device according to Claim 1,
wherein the sheet portion can be deformed between an open form in which the sheet portion is expanded in a direction that intersects a longitudinal direction of the elongated portion and a closed form in which the sheet portion is folded so as to be placed along the longitudinal direction of the elongated portion, and
wherein an opening/closing mechanism that causes the sheet portion to be deformed between the open form and the closed form is provided.

3. A medical device according to Claim 1 or 2, wherein the notifying portion is provided with a color layer that is provided in a surface of the sheet portion, which is disposed facing the surface of the body wall, and that has a color that is complementary to the color of the body wall or a color that is close to the complementary color.

4. A medical device according to any one of Claims 1 to 3, wherein the attaching portion protrudes farther than a surface, which is disposed facing a surface of the body wall, of the sheet portion does.

5. A medical device according to Claim 4, wherein the attaching portion is formed of a biological adhesive that is applied around the entire circumference of the periphery of the sheet portion.

6. A medical device according to Claim 4,
wherein the attaching portion is provided with a suction port provided around a circumferential direction thereof, and
wherein a suction device that is disposed at a proximal end of the elongated portion and that generates a suction force, and
a suction pipe that connects the suction port and the suction device via the elongated portion are provided.

7. A medical device according to any one of Claims 1 to 6, wherein the notifying portion is provided with a reflector that is provided in a surface of the sheet portion, which is disposed facing a surface of the body wall, and that reflects light.

8. A medical device according to any one of Claims 1 to 7, wherein the notifying portion is provided with a light-emitting body that is provided on a surface of the sheet portion, which is disposed facing a surface of the body wall, and that emits light.

9. A medical device according to any one of Claims 1 to 8, wherein the notifying portion is provided with:
a contact detecting means that is provided on the sheet portion and that detects contact with a treatment tool used to make an incision in the body wall; and
a recognizing means for visually or aurally making the surgeon recognize that contact with the treatment tool has been detected by the contact detecting means.

10. A medical device according to any one of Claims 1 to 9, wherein the sheet portion is provided with an insulating layer possessing electrical insulating properties.

11. A medical device according to any one of Claims 1 to 10,
wherein the sheet portion is provided with a conductive layer possessing conductivity, and
wherein an earth line that connects the conductive layer to earth via the elongated portion is provided.
